# FASCICULE DE BREVET EUROPEEN

(11) **EP 0 336 822 B1**
(45) Date de publication et mention de la délivrance du brevet: **10.08.1994**
(21) Numéro de dépôt: 89400890.3
(22) Date de dépôt: 30.03.1989
(51) Int. Cl.: C12N 15/86

(54) **Rétrovirus recombinant défectif, son application à l'intégration de séquences codantes pour des protéines déterminées dans le génome de cultures cellulaires infectables par le rétrovirus sauvage correspondant et ADNs recombinants pour la production de ce rétrovirus recombinant**
Defektives rekombinantes Retrovirus, seine Verwendung zur Integration von Proteine codierenden Sequenzen im Genom von Gewebekulturen, infizierbar mit dem entsprechenden Wildretrovirus, und DNS zur Herstellung dieses rekombinanten Retrovirus
Defective recombinant retrovirus,its use for integrating protein coding sequences in the genome of cell culture which can be infected by corresponding wild-type retrovirus,and recombinant DNA for obtaining this recombinant retrovirus

(30) Priorité: 31.03.1988 FR 8804332
(43) Date de publication de la demande: 11.10.1989
(73) Titulaire: INSTITUT PASTEUR, 75724 Paris Cédex 15 (FR); INSTITUT NATIONAL DE LA SANTE ET DE LA RECHERCHE MEDICALE (INSERM), 75654 Paris Cédex 13 (FR); CENTRE NATIONAL DE LA RECHERCHE SCIENTIFIQUE (CNRS), 75700 Paris Cedex 07 (FR)
(72) Inventeur: Heidmann, Thierry, F-75017 Paris (FR); Nicolas, Jean-François, F-78590 Noisy (FR)
(74) Mandataire: Gutmann, Ernest

(56) Documents cités:
- EP-A- 0 162 319
- EP-A- 0 178 220
- EP-A- 0 237 157
- WO-A-86/00922
- BIOTECHNOLOGY, vol. 3, aout 1985, pp. 689-693, New York, USA; D. MCCORMICK: "Human Gene Therapy: The first round"
- PATENT ABSTRACTS OF JAPAN, vol. 11, no. 246, (C439), 11 aout 1987; & JP-A-62 051 991
- CHEMICAL ABSTRACTS, vol. 99, no. 1, 4 juillet 1983, pp. 163, 164, colonne 2, abrege no. 17374s, Columbus, Ohio, USA; S. M. ANDERSON et al: "Construction and isolation of a transforming murine retrovirus containing the SRC gene of Rous sarcoma virus"

## Description

L'invention concerne des moyens permettant l'intégration de séquences codantes pour des protéines déterminées dans le génome de cellules de lignées cellulaires eucaryotes, notamment de mammifères, plus particulièrement de lignées cellulaires infectables par un rétrovirus sauvage.

Plus particulièrement, l'invention concerne l'intégration de telles séquences codantes, dans des conditions telles qu'elles puissent être exprimées au sein même de ces cellules cellulaires infectées.

Dans sa forme finale, les moyens selon l'invention consistent en un rétrovirus recombinant défectif dérivé d'un rétrovirus sauvage ou natif capable d'infecter l'hôte cellulaire concerné, ce rétrovirus contenant les parties correspondant à celles qui, dans ce rétrovirus, agissent en "cis" du virus sauvage et assurent normalement l'empaquetage du rétrovirus, sa reverse-transcription en une molécule ADN et son intégration dans le génome de la cellule-hôte, ainsi que le site de polyadénylation de cet ARN, ce rétrovirus recombinant étant plus particulièrement caractérisé par la substitution à la partie délétée d'une séquence hybride de rétrotransposition comportant une séquence d'ADN codant pour un polypeptide déterminé sous le contrôle direct d'un promoteur intérieur à cette séquence hybride de rétrotransposition et reconnaissable par une polymérase de l'hôte cellulaire infectable par le susdit rétrovirus natif, cette séquence hybride de rétrotransposition étant elle-même placée sous le contrôle d'un promoteur extérieur à cette séquence hybride de rétrotransposition et inclus dans le génome du rétrovirus défectif, ce rétrovirus recombinant défectif étant caractérisé en ce que les positions relatives de la séquence codante à l'intérieur de la séquence hybride de rétrotransposition et du promoteur extérieur sont telles que la transcription et l'expression de ladite séquence codante, sous le contrôle du promoteur intérieur, dans un hôte cellulaire infecté par ledit rétrovirus recombinant défectif, soit susceptible d'être effectuée dans une direction inverse vis-à-vis de la direction de transcription de la séquence hybride de transposition sous le contrôle dudit promoteur extérieur.

Le rétrovirus recombinant défectif ainsi défini peut être mis en oeuvre aux fins indiquées ci-dessus par un procédé comprenant l'infection avec ce rétrovirus recombinant de cellules de mammifères infectables par le rétrovirus natif correspondant et, le cas échéant, la récupération des cellules dans lesquelles la séquence codante est exprimée.

Du fait de son caractère défectif, le rétrovirus recombinant selon l'invention ne peut se répliquer dans ces cellules tout en conservant son caractère infectieux à leur égard. Il peut également s'intégrer sous forme de provirus dans le génome des cellules-hôtes. La séquence codante contenue dans la séquence hybride de rétrotransposition peut alors être exprimée sous le contrôle du "promoteur intérieur" à la séquence hybride de rétrotransposition.

Le promoteur intérieur peut être constitué par tout promoteur reconnu par les polymérases de l'hôte infectieux, par exemple un promoteur de SV40 ou de thymidine kinase, dans le cas de cellules appartenant à des lignées de cellules de mammifères supérieurs, par exemple humaines ou de hamsters (par exemple cellules CHO).

Un rétrovirus recombinant défectif conforme à un mode de réalisation préféré de l'invention est caractérisé en ce que le promoteur extérieur distinct est celui de la région LTR^{5′} du rétrovirus correspondant.

Plus particulièrement, le génome d'un rétrovirus recombinant défectif de ce type comprend successivement :
- la région LTR^{5′} du rétrovirus sauvage correspondant suivie de la région d'empaquetage ;
- le site d'initiation de l'ARN correspondant aux protéines virales normalement codées par les gènes gag, pol et env ;
- la séquence hybride de retransposition dans l'orientation sus-définie, en lieu et place de tout ou partie de la région rétrovirale comportant ces gènes gag, pol et env, cette séquence hybride de rétrotransposition étant placée sous le contrôle d'un promoteur extérieur approprié inclus dans le génome du rétrovirus, le cas échéant de la région LTR^{5′} elle-même ;
- la région LTR³, qui coïncide avec le site de polyadénylation du rétrovirus sauvage correspondant.

L'invention concerne plus particulièrement des ADNs recombinants (et parties d'ADNs recombinants) permettant la production des susdits rétrovirus recombinants défectifs, ainsi qu'un procédé de fabrication des rétrovirus recombinants susdits.

Au titre d'un ADN recombinant (ci-après désigné sous l'expression "ADN de rétrovirus recombinant défectif"), lui-même dérivé d'un rétrovirus sauvage ou natif utilisable pour la production du susdit rétrovirus recombinant défectif, on mentionnera plus paticulièrement un ADN contenant les parties correspondant à celles qui agissent en "cis" du virus sauvage et assurent normalement l'empaquetage de ce rétrovirus, sa reverse-transcription en une molécule ADN et son intégration dans le génome de la cellule-hôte, ainsi que le site de polyadénylation de cet ARN, cet ADN de rétrovirus recombinant étant plus particulièrement caractérisé par la délétion d'une région correspondant à au moins une partie sinon de la totalité des séquences codant pour les protéines virales du virus sauvage ou natif, la partie délétée étant suffisamment importante pour que soit interdite la réplication du rétrovirus défectif susceptible d'être produit dans une cellule-hôte compétente correspondante transformée avec cet ADN de rétrovirus recombinant défectif, celui-ci étant caractérisé par la substitution à la partie délétée d'un gène recombinant dont la structure est définie ci-après et qui est susceptible d'être transcrit sous le contrôle d'un promoteur distinct inclus dans l'ADN de rétrovirus recombinant, cependant extérieur vis-à-vis de ce gène recombinant.

Ce gène recombinant est plus particulièrement caractérisé par le fait qu'il contient une séquence codant pour le peptide déterminé recherché, celle-ci étant - à l'intérieur de ce gène recombinant - placée en aval d'un promoteur intérieur dans la direction de transcription imposée par ledit promoteur et en outre caractérisé par l'intercalation entre ledit promoteur intérieur et ladite séquence codante d'un fragment hybride comprenant lui-même une séquence apte à bloquer ladite transcription, cette séquence bloquante étant elle-même, au sein de ce fragment hybride, flanquée à ses deux extrémités opposées d'un site donneur d'épissage du côté de la séquence codante, d'une part, et d'un site accepteur d'épissage du côté du susdit promoteur, d'autre part. Les susdits éléments intercalaires interdisent par conséquent la transcription de la séquence codante sous le contrôle du promoteur intérieur susdit. En revanche lorsque ce gène recombinant est dans son entier placé sous le contrôle d'un promoteur extérieur distinct du précédent, dans des conditions telles que ce gène recombinant puisse (en l'absence de codon d'interruption) être transcrit dans une direction inverse (direction reverse) à celle imprimée par le promoteur intérieur, on obtient que dans un hôte cellulaire compétent le gène recombinant entier soit transcrit sous le contrôle du promoteur extérieur en un ARN messager dont est excisée la région intercalaire entre le promoteur intérieur et la séquence codante, c'est-à-dire la région contenant la séquence bloquante, flanquée à ses deux extrémités opposées des susdits sites donneurs et accepteurs d'épissage. Dans l'ARN messager obtenu, la séquence d'ARN correspondant à la séquence codant pour le polypeptide déterminé est alors placée sous le contrôle direct de la région correspondant à la région promotrice interne.

Ledit gène recombinant est inséré dans l'ADN de rétrovirus recombinant défectif, de façon telle que l'orientation de ce gène recombinant au sein de l'ADN rétroviral, d'une part, et les positions relatives de ce gène recombinant et du promoteur extérieur, d'autre part, soient telles que la transcription dudit gène recombinant sous le contrôle de ce promoteur extérieur soit susceptible d'être effectuée dans des cellules-hôtes d'assurer la production du rétrovirus recombinant défectif ainsi constitué dans une direction inverse de celle sous laquelle s'effectuerait la transcription de la séquence codante pour le peptide recherché, si elle n'en était pas empêchée par la séquence intercalée susdite.

L'invention concerne aussi le procédé de production d'un tel gène recombinant, ainsi que le procédé de production d'ADN de rétrovirus recombinant défectif.

Partant alors d'un tel ADN de rétrovirus recombinant défectif, il est possible de produire le susdit rétrovirus recombinant défectif par transfection d'une lignée cellulaire auxiliaire contenant, incorporées à son propre génome :
- une séquence nucléique (ci-après dénommée "séquence de complémentation") capable de suppléer à l'absence dans le vecteur recombinant défectif des séquences codant pour les protéines virales autorisant sa propagation dans les cellules qu'il est susceptible d'infecter ;
- la culture de la lignée cellulaire auxiliaire ainsi transfectée dans des conditions permettant la réplication de ce rétrovirus recombinant in situ, grâce à l'apport par complémentation "en trans" des protéines virales codées par la susdite "sequence de complémentation" ;
- la récupération du virus recombinant défectif produit dans le surnageant de culture de ladite lignée cellulaire auxiliaire.

En particulier, la séquence de complémentation contient plus particulièrement la partie de l'ADN génomique du rétrovirus sauvage correspondant, codant notamment pour les protéines virales, à savoir les gènes gag, pol et env du rétrovirus sauvage correspondant, dont le rétrovirus recombinant défectif est lui-même dépourvu.

La transcription du susdit gène recombinant, sous l'effet du promoteur extérieur contenu dans l'ADN de rétrovirus défectif, comme suite à une transfection d'un hôte cellulaire compétent, dans les conditions qui ont été indiquées plus haut, permet alors la fabriction du rétrovirus recombinant défectif correspondant, dans lequel la séquence codant pour le polypeptide déterminé se trouve placée sous le contrôle direct du promoteur intérieur sus-défini.

La "séquence bloquante" contenue dans le gène recombinant tel qu'il a été défini plus haut est avantageusement constituée par un site de polyadénylation . Il va de soi que toute autre séquence bloquante peut être utilisée. Elle peut être constituée par une séquence comportant plusieurs codons d'initiation et d'arrêt dans la direction de transcription imposée par le promoteur intérieur. Il va de soi que dans ce qui précède les enchaînements de nucléotides dont est formé ledit gène recombinant sont, le cas échéant, choisis de façon telle que la totalité du gène recombinant puisse être transcrit à partir d'un promoteur extérieur dans la susdite direction reverse.

Avantageusement le gène recombinant, tel qu'il a été défini plus haut, est placé dans les conditions sus-indiquées à l'intérieur de l'ADN rétroviral défectif, sous le contrôle d'une région promotrice correspondant à la région LTR^{5′} du rétrovirus sauvage. Un ADN rétroviral recombinant défectif préféré de l'invention comprend par conséquent et successivement :
- la région LTR^{5′} du rétrovirus sauvage correspondant, suivie de la région d'empaquetage ;
- le site d'initiation de l'ARN correspondant aux protéines virales normalement codées par les gènes gag, pol et env ;
- le gène recombinant susdéfini dans l'orientation sus-définie, en lieu et place de tout ou partie de la région rétrovirale comportant ces gènes gag, pol et env, ce gène recombinant étant placé sous le contrôle d'un promoteur extérieur approprié, le cas échéant de la région LTR^{5′} elle-même ; et
- la région LTR^{3′}, qui coïncide avec le site de polyadénylation de l'ARN du rétrovirus sauvage correspondant.

Dans l'une des applications préférées de l'invention, la séquence codante placée sous le contrôle du promoteur intérieur dans le gène recombinant susdit, code pour une protéine toxique à l'égard de cellules susceptibles d'être infectées par le rétrovirus. On appréciera qu'à l'occasion de la production du rétrovirus recombinant défectif dans les susdites lignées cellulaires auxiliaires, celles-ci seront en fait protégées contre la protéine toxique, en raison du blocage de la transcription de cette séquence codante sous l'effet du promoteur intérieur à ce gène recombinant, en raison de l'intercalation de la séquence de blocage. En revanche, des cellules infectables par le rétrovirus natif, et naturellement également par le rétrovirus recombinant défectif selon l'invention, peuvent devenir sensibles, voire être détruites, lorque ces cellules ayant été infectées avec le rétrovirus recombinant défectif, on assure l'intégration du provirus qui en résulte dans le génome desdites cellules et obtient l'expression de la séquence codante sous le contrôle du promoteur intérieur.

D'autres séquences codantes peuvent naturellement être incorporées dans le rétrovirus recombinant défectif ci-dessus décrit. Il s'agit par exemple de marqueurs. L'invention fournit alors dans ce cas une technique de marquage de cellules susceptibles d'être infectées par un rétrovirus correspondant à un rétrovirus sauvage déterminé, la séquence codante se comportant alors comme un "indicateur" de la possibilité de ces cellules d'être infectées.

Une autre application de l'invention s'adresse à la destruction sélective de cellules cancéreuses au sein d'un tissu sain, en particulier lorsque les celllules cancéreuses portent un antigène spécifique à ces cellules cancéreuses. Cette destruction sélective devient possible dès lors que l'on dispose d'anticorps reconnaissant, d'une part, les antigènes spécifiques de ces cellules cancéreuses et, d'autre part, des protéines virales du rétrovirus recombinant et enfin, lorsque ces anticorps ayant été fixés sur les antigènes et protéines virales en question, on les amène en contact étroit, avec possibilité d'internalisation du virus défectif dans les cellules cancéreuses, par l'intermédiaire d'une deuxième catégorie d'anticorps contenant les deux premiers types d'anticorps.

D'autres caractéristiques de l'invention apparaîtront encore au cours de la description qui suit d'essais visant à démontrer le cractère opérationnel de trétrovirus recombinants défectifs conformes à l'invention, et ce à l'aide des dessins, dans lesquels :
Les figures 1A et 1B montrent de façon schématique les structurations d'un gène recombinant conforme à l'invention et son insertion dans un vecteur rétroviral défectif.
La figure 2 représente schématiquement les étapes successives de l'intégration, sous forme de provirus, d'un rétrovirus recombinant défectif, obtenu à partir de l'ADN recombinant de la figure 1, dans une cellule infectée et les étapes de sa transcription en ARN messager et sa reverse-transcription, puis intégration dans le génome, de la cellule infectée.
Les figures 3 et 4 sont des représentations schématiques de fractionnement sur gel des différents fragments d'ADNs testés, après digestion des ADNs extraits des cellules infectées par diverses enzymes de restriction.

Les expérimentations dont la description suit ont ont été effectuées en mettant en oeuvre un provirus défectif dérivé du rétrovirus Moloney leucémique de souris, dont la structure est représentative de la plupart des rétrovirus capables d'infecter des cellules de mammifères supérieurs. L'homme du métier comprendra que les essais dont la description suit peuvent être étendus de la même façon à d'autres types de rétrovirus.

Une description plus détaillée des figures facilitera la compréhension de la description qui suit des essais qui suivent :

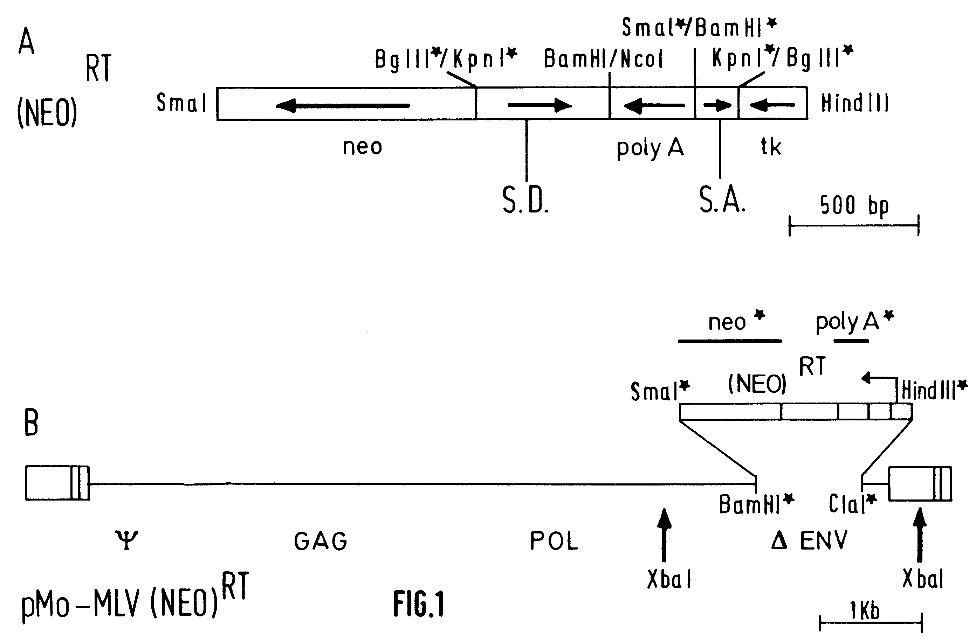

**figure 1** : Les éléments de base pour la détection de la rétrotransposition.
   (A) La structure de (néo) ^{RT}, un gène indicateur pour la rétrotransposition, y est représenté. Ce gène indicateur correspond au "gène recombinant" incorporé, destiné à être incorporé en lieu et place de l'une des régions qui, dans le génome du rétrovirus natif, code pour ses protéines structurales, en particulier le gène ENV. L'orientation de chaque élément génétique est indiquée par des flèches dans les bandes :
   tk représente le promoteur du gène de la thymidine kinase de HSV ;
   polyA représente la séquence de polyadénylation du gène précédemment cité ;
   neo représente la séquence du gène codant pour la résistance à la néomycine ;
   S.D. et S.A. représentent respectivement les sites d'épissage donneur et accepteur.
   En (B) est montrée la structure de pMo-MLV-(neo)^{RT} du rétrovirus recombinant, dans lequel le gène recombinant susdit a été incorporé ; la partie rétrovirale de la construction provient du provirus pMov3 cloné ; la figure montre également les fragments neo (fragment "indicateur" du rétrovirus recombinant dans lequel le gène recombinant susdit a été incorporé) et polyA (séquence de blocage).
   En (A) et (B) les sites de restriction utilisés pour la construction des plasmides sont marqués avec un astérisque lorsqu'ils sont éliminés.

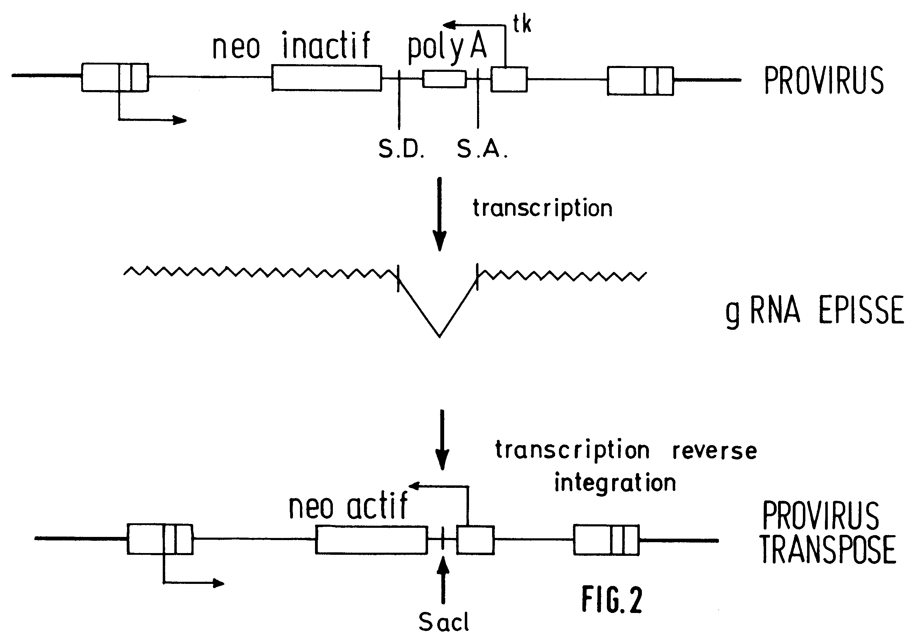

**La figure 2** représente les étapes successives de la production du rétrovirus défectif selon l'invention, impliquant le passage par une transcription de l'ADN rétroviral recombinant en ARN (ARNg épissé) pour aboutir au provirus qui s'intègre dans le génome d'une cellule infectée par le rétrovirus recombinant défectif.

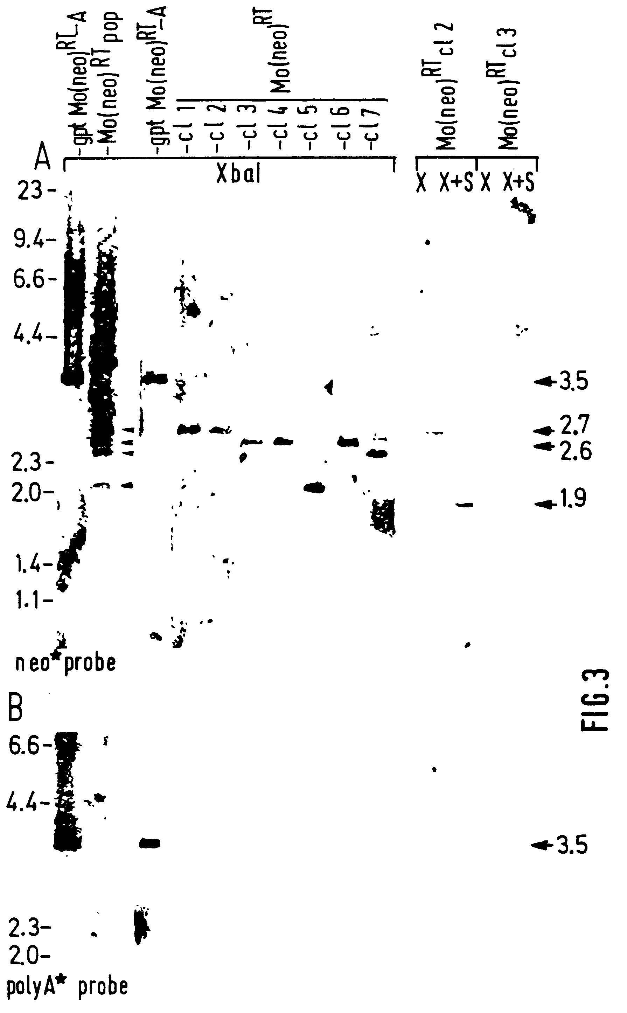

**Figure 3** : Analyse par la technique de "Southern blot" de l'ADN du genome des cellules G418-résistantes infectées avec le virus obtenu à partir de cellules transfectées avec pMo-MLV(neo)^{RT}.
   Les cellules provenant du clone 3T3gptMo(neo)^{RT}-A (un clone 3T3 obtenu par transfection de pSV2gpt et pMo-MLV(neo)^{RT} ont été infectées avec les virus Mo-MLV ; après sept jours le surnageant a été récupéré, et 100 ψl de ce surnageant ont été utilisés pour infecter 10⁴ cellules test 3T3 ; deux jours plus tard les cellules ont été soumises à une sélection en présence de G418. Les cellules G 418-résistantes ont été analysées soit sous forme d'une population de clones mélangés (population 3T3 Mo(neo)^{RT}), soit sous forme de clones individuels (3T3 Mo(neo)^{RT} cl1 à cl7).
   Les enzymes de restriction utilisées sont XbaI (X), ou XbaI+Sac1 (X+S). Elles ont permis de vérifier que l'épissage s'est correctement effectué. 10 ou 25 µg d'ADN ont été utilisés (deux premières bandes). Les filtres ont été hybridés avec, soit la sonde neo (fragment BglII-SmaI,A), soit la sonde polyA (fragment Sma1-NcoI,B).

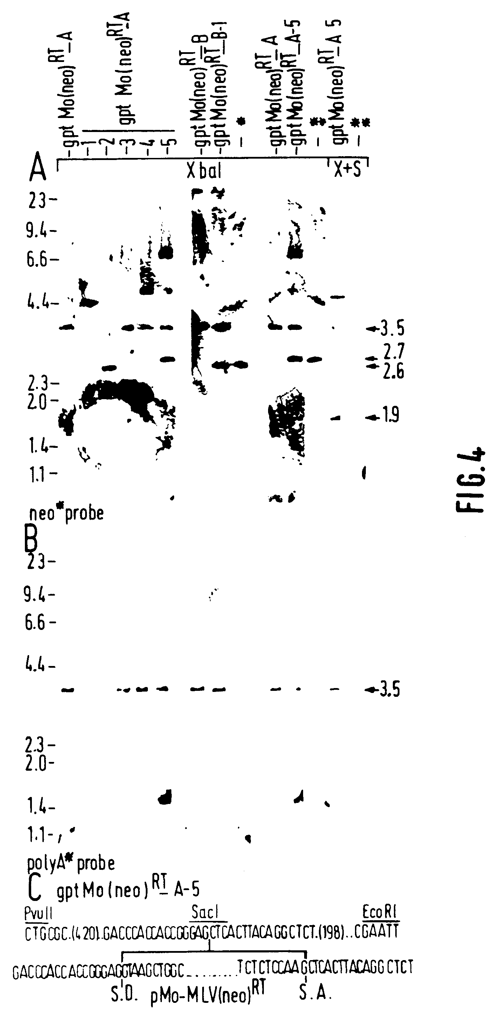

**Figure 4 :** Analyse par Southern blot de l'ADN du genome de cellules résistantes à G418 isolées à partir de clones transfectés avec 3T3gptMo(neo)^{RT} ; séquence d'une jonction d'épissage.
   (A et B) 10⁷ cellules provenant des clones 3T3gptMo(neo)^{RT} A et B ont été soumises à une sélection en présence de G 418 (10⁶ cellules / 10 cm de plaque). 0-2 clones résistant à G418 par plaque sont obtenus (voir par exemple 3T3 gptMo(neo)^{RT} clone A-1 à A-5, et clone cl1 B-1) ; l'ADN a été analysé comme décrit à propos de la figure 2, après restriction à l'aide de XbaI et XbaI+SacI afin de tester que l'épissage est correctement effectué. Les nouvelles copies dans le clone B-1 et dans le clone A-5 co-migrent avec respectivement les fragments XbaI de 2,6kb et de 2.7kb des clones Mo(neo)^{RT} cl3 (*) et cl2(**)de la figure 3. Les filtres ont été hybridés avec la sonde neo (A) ou la sonde polyA (B).
   (C) Jonction d'épissage dans une copie provirale transposée de pMo-MLV(neo)^{RT}.
   Le fragment XbaI de 2.7kb provenant de l'ADN de 3T3 gptMo(neo)^{RT}-A-5 a été cloné dans λgt10, et un fragment de 662 paires de bases, délimité par le site PvuII situé dans la région neo codante et par le site EcoRI situé dans le promoteur tk, a été sous-cloné dans M13 et séquencé. Le nouveau site SacI apparu au niveau de la jonction d'épissage est indiqué.

### 1) Matériels et méthodes

### a) Constructions des plasmides

Afin de construire l'indicateur de rétro-transposition (neo)^{RT}, la séquence codant pour la polyadénylation du gène de la thymidine kinase du virus Herpès Simplex a été isolée à partir du plasmide pAGO (COLBERE-GARAPIN, F. et al, J. Mol. Biol., 150, 1-14 (1981)) sous forme d'un fragment SmaI-NcoI de 310 paires de bases de longueur, et a été insérée au niveau de l'unique site BamHI du plasmide pZip(CEPKO, C. L., et al, Cell. 37, 1053-1062 (1984)), entre les sites d'épissage donneur et accepteur de Moloney, après traitement par l'enzyme de Klenow des deux fragments. Le fragment contenant la séquence de polyadénylation et les sites d'épissage, a ensuite été isolé à partir d'un fragment KpnI-KpnI, et inséré entre le promoteur SVtk et la région codante pour le gène (neo) au niveau du site BglII de pSVtk-neobeta (NICOLAS, J. S. et BERG, P., in Cold Spring Harbor Conferences on Cell Proliferation, eds. Silver, L.M. MARTIN, G.R & Strickland, S. S., 10, 469-485 (1983)), après traitement par l'enzyme de Klenow des deux fragments ; le gène indicateur neo^{RT} (fig. 1A) a été isolé à partir d'un fragment HindIII-SmaI.

De manière à supprimer les gènes env dans le plasmide pMov3 (HARBERS, K., et al, Proc. Natl. Acad. SCI., USA, 78 , 7609-7613 (1981)), le fragment EcoRI du plasmide pMVO3 à tout d'abord été recloné dans le fragment EcoRI de 3,4 kb du pSVtk-neobeta ; le fragment BamHI-ClaI (correspondant respectivement aux paires de bases 6537 et 7674 décrites dans RNA Tumor Viruses eds. WEISS, R. TEICH, N., VARMUS, H & COFFIN, J. , 2, 766-767 (1985)) codant pour une partie de la protéine gp70 et pour la protéine entière p15E a alors été éliminé par digestion complète à l'aide de ClaI et par digestion partielle à l'aide de BamHI.

La construction finale (pMo-MLV(neo)^{RT}) a été obtenue par ligature du vecteur pMov3, dont les gènes env ont éte supprimés de la manière indiquée ci-dessus, avec le gène indicateur (neo)^{RT} après traitement par l'enzyme de Klenow (fig. 1B).

### b) Culture de cellules, transfection et infection

Ces méthodes sont décrites dans JACOB, H. et NICOLAS , J.S., in Methods in Enzymol., 151, 66-81 (1987)). Les cellules utilisées sont NIH 3T3 et FG10 (FINSHINGER et al, Virology, 59, 217-229 (1974)), et les lignées virales utilisées sont la 3T3 Mo-MLV pour Moloney et E40psiB pour Mo-MLVneo (RUBEINSTEIN, J.L.R. et al, Proc. Natl. Acad. Sci., USA, 81, 7137-7140 (1984)). La sélection des cellules résistantes G418 a été réalisée en présence de 600 µg/ml de geneticine (G418), et les cellules contenant pSV2gpt (MULLIGAN, R.C. et al, Proc. Natl. Acad. Sci., USA, 78, 2072-2076 (1981)) ont été sélectionnées en présence de 25 µg/ml d'acide mycophénolique et 250 µg/ml de Xanthine.

Les infections ont été réalisées comme il est décrit dans JACOB., H et NICOLAS, J.F. (1987) précédemment cité en présence de 5 µg/ml de polybrène.

### c) Analyse de l'ADN des cellules

Pour l'analyse de Southern, l'ADN génomique traité par des enzymes de restriction appropriées, a été soumis à une électrophorèse, transféré sur des membranes de nylon Hybond-N (AMERSHAM), et hybridé à des sondes marquées radioactivement à l'aide de p³² (kit MULTIPRIME commercialisé par AMERSHAM).

### d) Clonage et séquencage de la jonction du site d'épissage

50 µg de l'ADN provenant du clone 3T3 gptMo(neo)^{RT}-A-5 a été digéré avec XbaI et soumis à une électrophorèse dans un gel d'agarose à 0,7 %. Une bande d'agarose comprenant des fragments de 2,5 à 2,9 kb à été découpée, et l'ADN extrait par électroélution a été utilisé pour construire une banque génomique partielle dans le vecteur λgt 10, selon des procédés standards et en ayant recours à des adaptateurs (linkers) EcoRI (HUYNH et al, DNA Cloning, ed. Glover, D.M., N.Y., IRL Press, 1, 49-78 (1985)). Les clones ont été sélectionnés par hybridation avec la sonde neo marquée au p³² sur un filtre support. Des fragments PvuII-EcoRI provenant de clones positifs ont été sous-clonés dans le vecteur M13mp10 et séquencés par la méhtode décrite dans SANGER, F. et al. (Proc. Natl. Acad. Sci., USA, 74, 5463-5467 (1977)).

### 2) Résultats

### a) Principe du test

Le principe du test repose sur l'utilisation d'un gène de sélection qui peut être activé par une transposition médiée par un ARN (fig. 1). Un gène (tkneo) est inactivé en insérant entre le promoteur (tk) et la partie structurelle du gène, un signal et un site pour la polyadénylation (polyA) ; la séquence pour la polyadénylation est entourée sur le brin d'ADN non codant par les sites d'épissage donneur et accepteur (neo^{RT}, fig. 1A). Lorsque la séquence polyA est insérée suivant une orientation opposée dans un rétro-transposon (pMo-MLV(neo)^{RT}, fig. 1B), la transcription réverse de l'ARN neo^{RT} épissé non codant conduit à la formation d'un ADN de structure dans lequel le gène neo est placé sous le contrôle du promoteur tk. Une cellule contenant une copie rétro-transposée devient par conséquent résistante à G418 (fig. 1C).

Dans le gène neo^{RT} (1) l'expression du gène sélectionné doit subsister tant que la région polyA est présente dans la séquence intronique et (2) la suppression de la région polyA devrait créer une structure conférant à une cellule hôte une résistance à G418.

Ces deux derniers points et la rétro-transposition non virale des rétrovirus ont été testés par insertion du gène neo^{RT} dans un provirus Mo-MLV cloné et dont les gène codant pour les composants majeurs de l'enveloppe virale ont été supprimés.

### b) pMo-MLV(neo) ^{RT} ne confère pas de résistance à G418

Afin de prouver que (neo)^{RT} satisfait aux deux points précédemment cités, il a été introduit dans des cellules NIH 3T3 par transfection de pMo-MLV(neo)^{RT}. pSV2gpt a été co-transfecté de manière a isoler des clones dans lesquels pMo-MLV(neo)^{RT} n'est pas sélectivement introduit (clones 3T3 gptMo(neo)^{RT}, et de manière à mesurer les efficacités de transfection.

Dans un premier test d'inactivation du gène neo dans neo^{RT}, la population de cellules transfectées a été directement soumise à une sélection dans un milieu contenant l'antibiotique G418, et le nombre de clones résistants a été comparé à ceux obtenus par transfection d'un plasmide dans lequel le gène neo est actif (pMo-MLVneo, RUBENSTEIN, J.L.R. et al (1984) précédemment cité). pMo-MLV(neo)^{RT} ne permet que l'obtention de 0-3 clones résitants à G418 par transfection (3 expériences), alors que plus de 75 clones résistants à G418 sont obtenus avec le plasmide pMo-MLVneo (une sélection parallèle dans un milieu contenant un acide mycophénolique indique que les efficacités de transfection sont identiques (tableau 1)).

L'analyse du type Southern de la structure de 5 clones individuels résistants à G418 indique la présence de ré-arrangements (probablement dus à la transfection) d'un fragment XbaI de 3,5 kb qui contient le gène indicateur (indiqué à la fig. 1a).

Dans un second test, les clones ont été sélectionnés par l'expression de gpt, et 7 d'entre eux contenant un gène (neo)^{RT} non ré-arrangé (voir par exemple les clones 3T3gptMo(neo)^{RT}-A et -B, de la fig. 3) ont été soumis à G418: aucun d'entre eux ne sont résistants à G418 (voir également les paragraphes suivants).

Ces expériences montrent que le gène (neo)^{RT} ne confère pas de résistance à G418, à moins qu'il ne soit ré-arrangé.

### c) Séquence résultant de la transcription réverse de pMo-MLV(neo) ^{RT} confère une résistance à G418

Une fraction de l'ARN produit par pMo-MLV(neo)^{RT} dans les cellules transfectées doit être épissé, et par conséquent ne contient pas la séquence de polyadénylation. Cet ARN conserve les caractéristiques d'un ARN d'un génome viral, qui par un mécanisme de trans-complémentation de produits viraux doit être empaqueté à l'intérieur de particules virales. Afin de tester si les copies obtenues par transcription réverse (fig. 2) peuvent conférer une résistance à G418, le surnageant des cellules 3T3gptMo(neo)^{RT} infectées avec le virus Mo-MLV a été tranféré aux cellules test NIH 3T3.

Des colonies résistantes à G418 ont été obtenues à partir de surnageants de 7 clones 3T3gpt(neo)^{RT} transfectés sus-mentionnés. Le titre des surnageants varie de 0,5 à 2 x 10³ neo^{R}fu/ml. La structure des nouveaux provirus a été analysée par la méthode de Southern (Southern blot).

Comme il est décrit dans la figure 3a, le fragment XbaI contenant (neo)^{RT} observé dans les clones 3T3 gptMo(neo)^{RT} avec la sonde neo a une taille réduite dans la population de cellules infectées (population Mo(neo)^{RT}) ou dans les sous-clones (Mo(neo)^{RT} c11 à c17). On peut observer jusqu'à quatre bandes d'intensités inégales dans la population, deux d'entre elles (de 2,7 et 2,6 kb) étant majoritairement représentées dans les sous-clones. Sur 25 clones infectés et analysés, 8 contiennent la bande de 2,7 kb et 12 la bande de 2,6 kb. Les deux autres bandes observées sont plus petites (2,5 kb et 2,0 kb), et la bande originale 3,5 kb n'a jamais été observée. Aucune de ces quatre bandes ne s'hybrident avec la sonde polyA du domaine intronique (fig. 3b).

La bande de 2,7 kb a été plus particulièrement étudiée afin de déterminer si elle contient le site SacI qui devrait apparaître au site de jonction d'épissage (MANN, R. et al, J. Virol., 54, 401-407 (1985); fig. 2). Le fragment de 2,7 kb est effectivement réduit à un fragment de 1,9 kb lorsque l'ADN génomique est digéré par les enzymes de restriction XbaI et SacI et soumis à un test d'hybridation en utilisant la sonde neo. Au contraire la bande de 2,6 kb ne contient pas de site sacI, et doit correspondre à des évènements d'épissage impliquant des sites cryptiques D-A ou à des réarrangements durant la transcription réverse.

En conclusion, pMo-MLV(neo)^{RT} permet le passage, par l'intermédiaire d'un ARN intermédiaire, à des structures dans lesquelles le gène neo est actif.

### d) Rétrotransposition du provirus Moloney env⁻

10⁸ cellules provenant de quatre clones 3T3gpt(neo)^{RT} indépendants ont été soumises à la sélection G418. Dans chaque cas, 1 à 10 clones résistants, sur 10⁷ cellules, ont été obtenus, et leur ADN a été analysé (fig. 4). Ils peuvent être regroupés en deux catégories.

Dans la première (13 clones parmi 37 clones résistants à G418), le fragment XbaI de 3,5 kb (correspondant au plasmide transfecté de la fig. 1A) est remplacé par un nouveau fragment (fig. 3, c11 et c12), qui ne s'hybride pas avec la sonde intronique polyA (fig. 3B) ; ces clones peuvent donc être considérés comme des réarrangements des copies initiales dans lesquelles au moins la séquence polyA est supprimée.

Dans la seconde catégorie (24 clones parmi les 37 clones analysés), une et parfois deux bandes hybridant avec la sonde neo sont observées en plus de la bande initiale de 3,5 kb des clones transfectés (fig. 4A, clones A-3 à A-5, et B-1). Ces bandes additionnelles ne s'hybrident pas avec la sonde polyA (fig. 4B). Dans plusieurs cas, (respectivement 5 et 2 clones), ces bandes co-migrent soit avec la copie d'épissage de 2,6 kb, soit avec celle de 2,6 kb des clones infectés (fig. 4A, clones A-5, et B-1). La mise en évidence du fait que la bande de 2,7 kb provient de la transcription réverse d'une séquence de transcription correctement épissée du provirus transfecté a été réalisée par digestion de l'ADN par XbaI et SacI qui génère une bande de 1,9 kb (fig. 4A, clone A-5). Cette conclusion est confirmée par le clonage d'un fragment XbaI de 2,7 kb à partir d'un des clones G418 résistant à 64,8 (clone A-5) et par le séquençage du site de jonction d'épissage (fig. 4C).

Il résulte donc des essais qui précèdent que l'on peut obtenir un ARN recombinant qui, après retranscription reverse, produit un ADN recombinant apte à fournir un ARN messager contenant une séquence d'ARN correspondant à la séquence neo qui peut à son tour être traduite sous le contrôle du promoteur intérieur qui lui est alors associé, à partir du gène neo^{RT} dans un rétrovirus défectif (se comportant comme un rétrotransposon) dans une orientation telle que les sites d'épissage soient contenus dans une séquence nucléique transcrite à partir d'un promoteur extérieur du rétrovirus dans une direction inverse de la direction normale de transcription du marqueur neo, sous le contrôle du promoteur interne de neo^{RT}.

Il est à remarquer que des lignées transcomplémentantes dans lesquelles le rétrovirus recombinant défectif de l'invention pouvant être produites sont disponibles à l'American Type Culture Collection (ATCC) sous la désignation ψ2.

## Revendications

1. Gène recombinant comportant une séquence codante pour un polypeptide déterminé, placée en aval d'un promoteur intérieur dans la direction de transcription imposée par ledit promoteur dans un hôte cellulaire eucaryote compétent, notamment de mammifère, caractérisé par l'intercalation entre ledit promoteur et ladite séquence codante d'un fragment hybride comprenant une séquence apte à bloquer ladite transcription, cette séquence bloquante étant elle-même, au sein de ce fragment hybride, flanquée à ses deux extrémités opposées d'un site donneur d'épissage du côté de la séquence codante, d'une part, et d'un site accepteur d'épissage du côté du susdit promoteur, d'autre part, dans des conditions permettant l'excision de ce fragment hybride dans l'hôte cellulaire compétent, et ce lorsque ledit gène recombinant est placé sous le contrôle d'un promoteur extérieur distinct du précédent, également reconnaissable par les polymérases dudit hôte cellulaire compétent et ce dans des conditions permettant la transcription de l'ensemble des éléments définis ci-dessus dans une direction opposée (direction reverse) à celle imprimée par le susdit promoteur intérieur.

2. Gène recombinant selon la revendication 1 caractérisé en ce que la séquence apte à bloquer la première transcription susmentionnée est constituée par un site de polyadénylation.

3. ADN de rétrovirus recombinant défectif dérivé d'un rétrovirus sauvage ou natif apte à infecter un hôte cellulaire eucaryote compétent, notamment de mammifère, cet ADN contenant les parties correspondant à celles qui, dans ce rétrovirus, agissent en "cis" du virus sauvage et assurent normalement l'empaquetage du rétrovirus, sa reverse-transcription en une molécule ADN et son intégration dans le génome de la cellule-hôte, ainsi que le site de polyadénylation de cet ARN, cet ADN de rétrovirus recombinant étant plus particulièrement caractérisé par la délétion d'une région correspondant à au moins une partie sinon de la totalité des séquences codant pour les protéines virales du virus sauvage ou natif, la partie délétée étant suffisamment importante pour que soit interdite la réplication du rétrovirus défectif susceptible d'être produit dans une cellules-hôte compétente correspondante transformée avec cet ADN de rétrovirus recombinant défectif, celui-ci étant caractérisé par la substitution à la partie délétée du gène recombinant de la revendication 1 ou de la revendication 2, sous le contrôle d'un promoteur extérieur distinct inclus dans l'ADN de rétrovirus recombinant, les positions relatives de ce promoteur extérieur distinct et du gène recombinant, ainsi que l'orientation de celui-ci étant telles que la transcription dudit gène recombinant sous le contrôle de ce promoteur extérieur soit susceptible d'être effectuée dans ladite direction reverse, dans des cellules-hôtes infectables par le rétrovirus recombinant défectif ainsi constitué.

4. ADN recombinant rétroviral selon la revendication 3, caractérisé en ce que le promoteur extérieur distinct est celui de la région LTR^{5′} du rétrovirus.

5. ADN rétroviral recombinant défectif dont le génome comprend successivement :
- la région LTR^{5′} du rétrovirus sauvage correspondant, suivie de la région d'empaquetage ;
- le site d'initiation de l'ARN correspondant aux protéines virales normalement codées par les gènes gag, pol et env ;
- le gène recombinant de la revendication 1 ou 2 dans l'orientation sus-définie, en lieu et place de tout ou partie de la région rétrovirale comportant ces gènes gag, pol et env, ce gène recombinant étant placé sous le contrôle d'un promoteur extérieur approprié, le cas échéant de la région LTR^{5′} elle-même ; et
- la région LTR^{3'}, qui coincide avec le site de polyadénylation de l'ARN du rétrovirus sauvage correspondant.

6. Rétrovirus recombinant défectif dérive d'un rétrovirus sauvage ou natif capable d'infecter un hôte cellulaire eucaryote compétent, notamment de mammifère, ce rétrovirus contenant les parties correspondant à celles qui, dans ce rétrovirus, agissent en "cis" du virus sauvage et assurent normalement l'empaquetage du rétrovirus, sa reverse-transcription en une molécule ADN et son intégration dans le génome de la cellule-hôte, ainsi que le site de polyadénylation de cet ARN, ce rétrovirus recombinant étant plus particulièrement caractérisé par la substitution à la partie délétée d'une séquence hybride de rétrotransposition comportant une séquence d'ADN codant pour un polypeptide déterminé sous le contrôle direct d'un promoteur intérieur à cette séquence hybride de rétrotransposition et reconnaissable par une polymérase de l'hôte cellulaire infectable par le susdit rétrovirus natif, cette séquence hybride de rétrotransposition étant elle-même placée sous le contrôle d'un promoteur extérieur à cette séquence hybride de rétrotransposition, où il y a l'intercalation entre ledit promoteur intérieur et ladite séquence codante d'un fragment hybride comprenant lui-même une sequence apte à bloquer ladite transcription, cette séquence bloquante étant elle-même au sein de ce fragment hybride, flanquée à ses deux extrémités opposées d'un site donneur d'épissage du côte de la séquence codante, d'une part, et d'un site accepteur d'épissage du côte du susdit promoteur d'autre part, et inclus dans le génome du rétrovirus recombinant défectif étant caractérisé en ce que les positions relatives de la séquence codante à l'intérieur de la séquence hybride de rétrotransposition et du promoteur extérieur sont telles que la transcription et l'expression de ladite séquence codante sous le contrôle du promoteur intérieur dans un hôte cellulaire infecté par ledit rétrovirus recombinant soit susceptible d'être effectuée dans une direction inverse vis-à-vis de la direction de transcription de la séquence hybride de rétrotransposition sous le contrôle dudit promoteur extérieur.

7. Rétrovirus recombinant défectif selon la revendication 6, caractérisé en ce que le promoteur extérieur distinct est celui de la région LTR^{5'} du rétrovirus correspondant.

8. Rétrovirus recombinant défectif selon la revendication 7, dont le génome comprend successivement :
- la région LTR^{5′} du rétrovirus sauvage correspondant suivie de la région d'empaquetage;
- le site d'initiation de l'ARN correspondant aux protéines virales normalement codées par les gènes, gag, pol et env;
- la séquence hybride de rétrotransposition dans l'orientation sus-définie, en lieu et place de tout ou partie de la région rétrovirale comportant ces gènes gag, pol et env, cette séquence hybride de rétrotransposition étant placée sous le contrôle d'un promoteur extérieur approprié inclus dans le génome du rétrovirus, le cas échéant de la région LTR^{5′} elle-même;
- la région LTR³, qui coïncide avec le site de polyadénylation du rétrovirus sauvage correspondant.

9. Procédé de production d'un gène recombinant comportant une séquence codante pour un polypeptide déterminé, placée en aval d'un promoteur intérieur dans la direction de transcription imposée par ledit promoteur dans un hôte cellulaire eucaryote compétent, notamment de mammifère, caractérisé en ce qu'on intercale entre ledit promoteur et ladite séquence codante un fragment hybride comprenant une séquence apte à bloquer ladite transcription, cette séquence bloquante étant elle-même, au sein de ce fragment hybride, flanquée à ses deux extrémités opposées d'un site donneur d'épissage du côté de la séquence codante, d'une part, et d'un site accepteur d'épissage du côté du susdit promoteur, d'autre part, dans des conditions permettant l'excision de ce fragment hybride dans l'hôte cellulaire compétent, et ce lorsque ledit gène recombinant est placé sous le contrôle d'un promoteur extérieur distinct du précédent, également reconnaissable par les polymérases dudit hôte cellulaire compétent et ce dans des conditions permettant la transcription de l'ensemble des éléments définis ci-dessus dans une direction opposée (direction reverse) à celle imprimée par le susdit promoteur intérieur.

10. Procédé selon la revendication 9, caractérisé en ce que la séquence apte à bloquer la première transcription susmentionnée est constituée par un site de polyadénylation.

11. Procédé de production d'ADN de rétrovirus recombinant défectif derivé d'un rétrovirus sauvage ou natif apte à infecter un hôte cellulaire eucaryote compétent, notamment de mammifère, cet ADN contenant les parties correspondant à celles qui, dans ce rétrovirus, agissent en "cis" du virus sauvage et assurent normalement l'empaquetage du rétrovirus, sa reverse-transcription en une molécule ADN et son intégration dans le génome de la cellule-hôte, ainsi que le site de polyadénylation de cet ARN, caractérisé en ce qu on supprime une région correspondant à au moins une partie sinon de la totalité des séquences codant pour les protéines virales du virus sauvage ou natif, la partie délétée étant suffisamment importante pour que soit interdite la réplication du rétrovirus défectif susceptible d'être produit dans une cellule-hôte compétente correspondante transformée avec cet ADN de rétrovirus recombinant défectif, et en ce que ensuite on substitue à la partie délétée le gène recombinant produit selon la revendication 9 sous le contrôle d'un promoteur extérieur distinct inclus dans l'ADN de rétrovirus recombinant, les positions relatives de ce promoteur extérieur distinct et du gène recombinant, ainsi que l'orientation de celui-ci étant telles que la transcription dudit gène recombinant sous le contrôle de ce promoteur extérieur soit susceptible d'être effectuée dans ladite direction reverse, dans des cellules-hôtes infectables par le rétrovirus recombinant défectif ainsi constitué.

12. Procédé selon la revendication 11, caractérisé en ce que le promoteur extérieur distinct est celui de la région LTR^{5′} du rétrovirus.

13. Procédé pour la production d'un ADN rétroviral recombinant défectif dont le génome comprend successivement :
- la région LTR^{5′} du rétrovirus sauvage correspondant, suivie de la région d'empaquetage ;
- le site d'initiation de l'ARN correspondant aux protéines virales normalement codées par les gènes gag, pol et env ;
- le gène recombinant produit par le procédé des revendications 9 ou 10 dans l'orientation sus-définie, en lieu et place de tout ou partie de la région rétrovirale comportant ces gènes et gag, pol et env, ce gène recombinant étant placé sous le contrôle d'un promoteur extérieur approprié, le cas échéant de la région LTR^{5′} elle-même; et
- la région LTR^{3′}, qui coïncide avec le site de polyadénylation de l'ARN du rétrovirus sauvage correspondant, caractérisé en ce qu'on délete, d'un vecteur contenant l'ADN rétrovirale, toute ou une partie de la région comportant les gènes gag, pol et env, ensuite on substitue le gène recombinant dans la région déletée, cette substitution étant effectuée d'une manière qui place ce gène recombinant sous le contrôle d'un promoteur extérieur approprié.

14. Procédé pour l'incorporation d'une séquence de nucléotides codant pour un polypeptide déterminé dans le génome de cellules d'une culture cellulaire infectable par le rétrovirus natif correspondant au rétrovirus recombinant défectif, selon l'une quelconque des revendications 6 à 8, caractérisé par la mise en contact de ladite culture cellulaire avec ce rétrovirus recombinant défectif dans lequel la susdite séquence codante correspond à la susdite séquence de nucléotides codant pour le polypeptide déterminé, dans des conditions autorisant l'infection de ces cellules par le rétrovirus recombinant défectif et, le cas échéant, par l'isolement des cellules dans lesquelles la séquence codante est exprimée.

15. Procédé pour la production d'un rétrovirus recombinant défectif, selon l'une quelconque des revendications 6 à 8, comprenant :
- la transfection d'une lignée cellulaire auxiliaiaire avec un vecteur contenant l'ADN de rétrovirus recombinant défectif produit selon l'une des revendicationations 11 à 13, cette lignée cellulaire auxiliaire contenant, incorporée à son propre ADN génomique, une séquence nucléique (ci-après dénommée "séquence de complémentation") capable de suppléer à l'absence dans le vecteur recombinant défectif des séquences codant pour les protéines virales autorisant sa propagation dans les cellules qu'il est susceptible d'infecter ;
- la culture de la lignée cellulaire auxiliaire ainsi transfectée dans des conditions permettant la réplication de ce rétrovirus recombinant in situ, grâce à l'apport par complémentation "en trans" des protéines virales codées par la susdite "séquence de complémentation" ;
- la récupération du virus recombinant défectif produit dans le surnageant de culture de ladite lignée cellulaire auxiliaire.

16. Procédé selon la revendication 15, caractérisé en ce que la séquence de complémentation contient plus particulièrement la partie de l'ADN génomique du rétrovirus sauvage correspondant, codant notamment pour les protéines virales, à savoir les gènes gag, pol et env du rétrovirus sauvage correspondant, dont le rétrovirus recombinant défectif est lui-même dépourvu.

## Claims

1. Recombinant gene comprising a sequence coding for a specific polypeptide, placed downstream from an internal promoter in the direction of transcription imposed by said promoter in a competent, eukaryotic, in particular mammalian, cell host characterized by the intercalation between said promoter and the said coding sequence of a hybrid fragment comprising a sequence capable of blocking the said transcription, this blocking sequence being itself flanked at its two opposite ends, within this hybrid fragment, by a donor splicing site on the side of the coding sequence and by an acceptor splicing site on the side of the above-mentioned promoter under conditions allowing the excision of this hybrid fragment in the competent cell host when said recombinant gene is placed under the control of an external promoter distinct from the former but which can also be recognized by the polymerases of said competent cell host under conditions allowing the transcription of all of the elements defined above in the revers direction to that imposed by the above-mentioned internal promoter.

2. Recombinant gene according to Claim 1 characterized in that the sequence capable of blocking the first transcription mentioned above is constituted by a polyadenylation site.

3. DNA of defective recombinant retrovirus derived from a native or wild-type retrovirus capable of infecting a competent eukaryotic, in particular mammalian, cell host, this DNA containing the parts corresponding to those which, in this retrovirus, are "cis" acting in the wild-type virus and are normally responsible for the packaging of the retrovirus, its reverse transcription into a molecule of DNA and its integration into the genome of the host cell, as well as for the polyadenylation site of this RNA, this recombinant retroviral DNA being more particularly characterized by the deletion of a region corresponding to at least a part if not all of the sequences coding for the viral proteins of the native or wild-type virus, the deleted part being large enough to prevent the replication of the defective retrovirus capable of being produced in a corresponding competent host cell transformed by this defective recombinant retroviral DNA, the latter being characterized by the replacement of the deleted part by the recombinant gene of Claim 1 or Claim 2, under the control of a distinct external promoter included in the recombinant retroviral DNA, the relative positions of this distinct external promoter and of the recombinant gene as well as the orientation of this latter being such that the transcription of said recombinant gene under the control of this external promoter is capable of being carried out in said reverse direction in host cells which can be infected by the defective recombinant retrovirus thus constituted.

4. Retroviral recombinant DNA according to Claim 3, characterized in that the distinct external promoter is that of the LTR^{5'} region of the retrovirus.

5. Defective recombinant retroviral DNA whose genome comprises successively :
- the LTR^{5'} region of the corresponding wild-type retrovirus, followed by the packaging region;
- the initiation site of the RNA corresponding to the viral proteins normally encoded in the gag, pol and env genes;
- the recombinant gene of Claim 1 or 2 in the orientation defined above, in place of all or part of the retroviral region comprising these gag, pol and env genes, this recombinant gene being placed under the control of a suitable external promoter included in the retroviral genome, optionally the LTR^{5'} region itself; and
- the LTR³ region, which coincides with the polyadenylation site of the RNA of the corresponding wild-type retrovirus.

6. Defective recombinant retrovirus derived from a native or wild-type retrovirus capable of infecting a competent eukaryotic, in particular mammalian, cell host, this retrovirus containing the parts corresponding to those which, in this retrovirus, are "cis" acting in the wild-type virus and are normally responsible for the packaging of the retrovirus, its reverse transcription into a DNA molecule and its integration into the genome of the host cell, as well as for the polyadenylation site of this RNA, this recombinant retrovirus being more particularly characterized by the replacement of the deleted part by a hybrid retrotransposition sequence comprising a DNA sequence coding for a specific polypeptide under the direct control of an internal promoter within this hybrid retrotransposition sequence and which can be recognized by a polymerase of the cell host which can be infected by the above-mentioned native retrovirus, the hybrid retrotransposition sequence being itself placed under the control of a promoter external to this hybrid retrotransposition sequence, in which has been intercalated between said internal promoter and said coding sequence a hybrid fragment itself comprising a sequence capable of blocking the said transcription, this blocking sequence being itself flanked at its two opposite ends, within this hybrid fragment, by a donor splicing site on the side of the coding sequence and by an acceptor splicing site on the side of the said promoter and included in the genome of the defective recombinant retrovirus being characterized in that the relative positions of the coding sequence within the hybrid retrotransposition sequence and the external promoter are such that the transcription and the expression of said coding sequence under the control of the internal promoter in a cell host infected by said recombinant retrovirus is capable of being carried out in the reverse direction with respect to the direction of transcription of the hybrid retrotransposition sequence under the control of the said external promoter.

7. Defective recombinant retrovirus according to Claim 6, characterized in that the distinct external promoter is that of the LTR^{5'} region of the corresponding retrovirus.

8. Defective recombinant retrovirus according to Claim 7, whose genome successively comprises :
- the LTR^{5'} region of the corresponding wild-type retrovirus, followed by the packaging region;
- the initiation site of the RNA corresponding to the viral proteins normally encoded in the gag, pol and env genes;
- the hybrid retrotransposition sequence in the orientation defined above in place of all or part of the retroviral region comprising these gag, pol and env genes, this hybrid retrotransposition sequence being placed under the control of a suitable external promoter included in the retroviral genome, optionally the LTR^{5'} region itself; and
- the LTR³ region, which coincides with the polyadenylation site of the RNA of the corresponding wild-type retrovirus.

9. Production process for a recombinant gene comprising a sequence coding for a specific polypeptide placed downstream from an internal promoter in the direction of transcription imposed by said promoter in a competent eukaryotic, in particular mammalian, cell host characterized in that between said promoter and said coding sequence a hybrid fragment is intercalated comprising a sequence capable of blocking said transcription, this blocking sequence being itself flanked at its two opposite ends, within this hybrid fragment, by a donor splicing site on the side of the coding sequence and by an acceptor splicing site on the side of the above-mentioned promoter under conditions which allow the excision of this hybrid fragment in the competent cell host and do so when said recombinant gene is placed under the control of an external promoter distinct from the former and which can also be recognized by the polymerases of said competent cell host under conditions allowing the transcription of all of the elements defined above in the reverse direction to that imposed by the above-mentioned internal promoter.

10. Process according to Claim 9, characterized in that the sequence capable of blocking the first transcription mentioned above is constituted by a polyadenylation site.

11. Production process for defective recombinant retroviral DNA derived from a native or wild-type retrovirus capable of infecting a competent eukaryotic, in particular mammalian, cell host, this DNA containing the parts corresponding to those which, in this retrovirus, are "cis" acting in the wild-type virus and are normally responsible for the packaging of the retrovirus, its reverse transcription into a DNA molecule and its integration into the genome of the host cell, as well as for the polyadenylation site of this RNA, characterized in that a region corresponding to at least a part if not all of the sequences coding for the viral proteins of the native or wild-type is deleted, the deleted part being large enough to prevent the replication of the defective retrovirus capable of being produced in a corresponding competent host cell transformed by this defective recombinant retroviral DNA, and in that the deleted part is then replaced by the recombinant gene produced according to Claim 9 under the control of a distinct external promoter included in the recombinant retroviral DNA, the relative positions of this distinct external promoter and the recombinant gene as well as the orientation of the latter being such that the transcription of said recombinant gene under the control of this external promoter can be carried out in said reverse direction in host cells which can be infected by the defective recombinant retrovirus thus constituted.

12. Process according to Claim 11, characterized in that the distinct external promoter is that of the LTR^{5'} region of the retrovirus.

13. Production process for a defective recombinant retroviral DNA whose genome comprises successively :
- the LTR^{5'} region of the corresponding wild-type retrovirus followed by the packaging region;
- the initiation site of the RNA corresponding to the viral proteins normally encoded in the gag, pol and env genes;
- the recombinant gene produced by the process of Claims 9 or 10 in the orientation defined above, in place of all or part of the retroviral region comprising these gag, pol and env genes, this recombinant gene being placed under the control of a suitable external promoter included in the retroviral genome, optionally the LTR^{5'} region itself;
- the LTR³ region, which coincides with the polyadenylation site of the RNA of the corresponding wild-type retrovirus, characterized in that all or part of the region comprising the gag, pol and env genes is deleted from a vector containing the retroviral DNA and then the recombinant gene replaces the deleted region, this replacement being carried out so as to place this recombinant gene under the control of a suitable external promoter.

14. Process for the incorporation of a nucleotide sequence coding for a specific polypeptide into the genome of cells of a cell culture infectable with the native retrovirus corresponding to the defective recombinant retrovirus according to any one of the Claims 6 to 8, characterized by the placing of the said cell culture in contact with this defective recombinant retrovirus in which the above-mentioned coding sequence corresponds to the above-mentioned nucleotide sequence coding for the specific polypeptide under conditions permitting the infection of these cells by the defective recombinant retrovirus and, optionally, by the isolation of the cells in which the coding sequence is expressed.

15. Production process for a defective recombinant retrovirus according to any one of the Claims 6 to 8, comprising :
- the transfection of a helper cell line with a vector containing the defective recombinant retroviral DNA produced according to any one of Claims 11 to 13, this helper cell line containing, incorporated in its own genomic DNA, a nucleic acid sequence (designated hereafter "complementation sequence") capable of compensating for the absence from the defective recombinant vector of the sequences coding for the viral proteins allowing its propagation in the cells which it is capable of infecting;
- culturing the helper cell line thus transfected under conditions making possible the replication of this recombinant retrovirus in situ, owing to the supply by means of "in trans" complementation of the viral proteins encoded in the above-mentioned "complementation sequence";
- the recovery of the defective recombinant virus produced in the culture supernatant of the said helper cell line.

16. Process according to Claim 15, characterized in that the complementation sequence contains more particularly the part of the genomic DNA of the corresponding wild-type retrovirus which codes in particular for the viral proteins, namely the gag, pol and env genes of the corresponding wild-type retrovirus, which the defective recombinant retrovirus itself lacks.

## Patentansprüche

1. Rekombinierendes Gen, das eine Kodiersequence für ein festgelegtes Polypeptid enthält, die in Transcriptaserichtung unterhalb eines inneren Promoters angeordnet ist und durch diesen Promoter in eine eukariotische zuständige Gastzelle eingeschleust ist, insbesondere eine Mammiferenzelle, **gekennzeichnet durch** das Einfügen eines Hybridfragments zwischen diesen Promoter und diese Kodiersequenz, wobei dieses Hybridfragment eine Sequenz enthält, die die Transcriptase blockieren kann und diese Blockersequenz selbst sich in der Mitte des Hybridfragments befindet und an ihren einander gegenüberliegenden Enden an einer Seite von einer impulsgebenden Stelle der Kodiersequenz und an der anderen Seite von einer impulsempfangenden Stelle des Promoters begrenzt ist, unter Bedingungen, die die Excision dieses Hybridfragments in der zuständigen Gastzelle erlauben, wobei das rekombinierende Gen der Kontrolle eines äußeren Promoters unterstellt ist, der sich vom vorherigen unterscheidet und ebenfalls von den Polymerasen der Gastzelle erkannt werden kann, und dieses unter Bedingungen, die die Transcription der Zusammenstellung der oben beschriebenen Elemente in eine entgegengesetzte Richtung (Gegenrichtung) zu der von dem inneren Promoter vorgegebenen Richtung erlauben.

2. Rekombinierendes Gen nach Anspruch 1, **dadurch gekennzeichnet**, daß die Sequenz, die die erste obengenannte Transcription blockieren kann, aus einer Stelle von Polyadenylation besteht.

3. ADN eines defektiven rekombinierenden Retrovirus, abgeleitet aus einem wilden oder natürlichen Retrovirus, der fähig ist, eine zuständige eukariotische Gastzelle, insbesondere eine Mammifere zu infizieren, wobei diese ADN diejenigen Abschnitte enthält, die denen entsprechen, die in diesem Retrovirus als "cis" des wilden Virus tätig sind und normalerweise die Umhüllung des Retrovirus, seine reverse Transcriptase in ein ADN-Molekül und seine Einbindung in das Genom der Gestzelle gewährleisten, ebenso wie die Steile der Polyadenylation dieses ADN, wobei diese ADN des rekombinierenden Retrovirus insbesondere genauer gekennzeichnet wird durch das Entfernen eines Gebiets, das wenigstens einem Teil, wenn nicht der Gesamtheit der Kodiersequenzen für die Virusproteine des wilden oder natürlichen Virus entspricht, wobei dieser entfernte Teil wichtig genug ist, um die Replikation des defektiven Retrovirus zu verhindern, und geeignet ist hergestellt zu werden in einer entsprechenden zuständigen Gestzelle, die mit der ADN des defektiven rekombinierenden Retrovirus transformiert worden ist, wobei dieser **gekennzeichnet** ist dadurch, daß er das entfernte Teil des rekombinierenden Gens nach Anspruch 1 oder 2 ersetzt, der Kontrolle eines äußeren bestimmten Promoters untersteht, der in der ADN des rekombinierenden Retrovirus eingeschlossen ist, wobei die gegenseitige Anordnung dieses äußeren bestimmten Promoters und des rekombinierenden Gens sowie dessen Orientierung so gewählt sind, daß die Transcription dieses rekombinierenden Gens unter der Kontrolle dieses äußeren Promoters in der erwähnten umgekehrten Richtung durchgeführt werden kann in den Gastzellen, die von dem so zusammengesetzten defektiven rekombinierenden Retrovirus infiziert werden können.

4. Rekombinierendes retrovirales ADN nach Anspruch 3, **dadurch gekennzeichnet**, daß der bestimmte äußere Promoter der Promoter aus dem Gebiet LTR5 des Retrovirus ist.

5. Rekombinierendes retrovirales defektives ADN dessen Genom nacheinander umfaßt:
- das Gebiet LTR5 des jeweiligen wilden Retrovirus, gefolgt von dem Gebiet der Umhüllung
- die Anfangsstelle des ARN, das den Virusproteinen zugeordnet ist, die normalerweise durch die Gene qaq, pol und env verschlüsselt sind
- das rekombinierende Gen nach Anspruch 1 oder 2 in der oben beschriebenen Ausrichtung, an Ort und Stelle des vollständigen oder unvollständigen Gebiets des Retrovirus, das die Gene qaq, pol und env umfaßt, wobei dieses rekombinierende Gen der Kontrolle eines geeigneten äußeren Promoters untersteht, der aus dem Gebiet LTR5 selbst hervorgeht, und
- das Gebiet LTR3, das zusammenfällt mit der Stelle der Polyadenylatopn des ADN des zugehörigen wilden Retrovirus.

6. Rekombinierender defektiver Retrovirus, abgeleitet von einem wilden oder natürlichen Retrovirus, der fähig ist, eine zuständige eukariotische Gestzelle, insbesondere eine Mammifere, zu infizieren, wobei dieser Retrovirus die Gebiete enthält, die denen entsprechen, die in diesem Retrovirus als "cis" des wilden Virus dienen und normalerweise die Umhüllung des Retrovirus, seine reverse Transcriptase in ein ADN-Molekül und seine Einbindung in das Genom der Gastzelle gewährleisten, ebenso wie die Stelle der Polyadenylation von diesem ADN, wobei dieser rekombinierende Retrovirus insbesondere gekennzeichnet ist dadurch, daß er den entfernten Bereich einer Hybridsequenz der Retrotransposition ersetzt, die eine ein bestimmtes Polypeptid verschlüsselnde ADN-Sequenz unter der Kontrolle eines Promoters innerhalb dieser Hybridsequenz umfaßt, der durch eine Polymerase der durch den oben genannten natürlichen Retrovirus infizierbaren Gastzelle erkennbar ist, wobei diese Hybridsequenz der Retrotransposition selbst der Kontrolle eines Promoters außerhalb dieser Hybridsequenz der Retrotransposition untersteht, wo es zu einer Intercalation zwischen diesem inneren Promoter und dieser ein Hybridfragment verschlüsselndes Sequenz kommt, wobei das Hybridfragment eine Sequenz umfaßt, die diese Transcription blockieren kann, wobei diese Blockiersequenz selbst sich in der Mitte des Hyridfragments befindet und an ihren einander gegenüberliegenden Enden an einer Seite von einer impulsgebenden Stelle aufder Seite der Kodiersequenz und an der anderen Seite von einer impulsempfangenden Stelle aufder Seite des obigen Promoters begrenzt ist, und eingeschlossen ist in das Genom des defektiven rekombinierenden Retrovirus, der **dadurch gekennzeichnet** ist, daß die gegenseitige Lage der Kodiersequenz im Inneren der Hybridsequenz der Retrotransposition und des äußeren Promoters so ist, daß die Transcription und die Ausbildung dieser Kodiersequenz unter der Kontrolle des inneren Promoters in einer von diesem rekombinierenden Retrovirus infizierten Gastzelle durchgeführt werden kann in einer Richtung entgegen der Richtung der Transcription der Hybridsequenz der Retrotransposition unter der Kontrolle dieses äußeren Promoters.

7. Rekombinierender defektiver Retrovirus nach Anspruch 6, **dadurch gekennzeichnet**, daß der bestimmte äußere Promoter derjenige des Gebiets LTR5 des zugehörigen Retrovirus ist.

8. Rekombinierender defektiver Retrovirus nach Anspruch 7, dessen Genom nacheinander umfaßt:
- das Gebiet LTR5 des zugehörigen wilden Retrovirus, gefolgt von dem Gebiet der Umhüllung
- die Anlangsstelle des ARN, das den Virusproteinen zugeordnet ist, die normalerweise von den Genen qaq, pol und env verschlüsselt werden,
- die Hybridsequenz der Retrotransposition in der oben beschriebenen Richtung, an Ort und Stelle des vollständigen oder unvollständigen Gebiets des Retrovirus, das die Gene qaq, pol und env umfaßt, wobei diese Hybridsequenz der Retrotransformation der Kontrolle eines geeigneten äußeren Promoters untersteht, der in dem Genom des Retrovirus eingeschlossen ist, der aus dem Gebiet LTR5 selbst hervorgeht,
- das Gebiet LTR3, das mit der Stelle der Polyadenylation des zugehörigen wilden Retrovirus zusammenfällt.

9. Verfahren zur Herstellung eines rekombinierenden Gens, das eine Kodiersequenz für ein bestimmtes Polypeptid enthält, die in Transcriptaserichtung unterhalb eines inneren Promoters angeordnet ist und durch diesen Promoter in eine zuständige eukariotische Gastzelle eingeschleust ist, insbesondere eine Mammifere, **dadurch gekennzeichnet**, daß zwischen diesen Promoter und diese Kodiersequenz ein Hybridfragment eingeschoben ist, das eine Sequenz enthält, die diese Transcription blockieren kann, wobei diese Blockiersequenz selbst sich in der Mitte des Hybridfragments befindet und an ihrer einander gegenüberliegenden Enden aufeiner Seite von einer impulsgebenden Stelle auf der Seite der Kodiersequenz und auf der anderen Seite von einer impulsempfangenden Stelle aufder Seite des Promoters begrenzt wird, unter Bedingungen, die die Excision dieses Hybridfragments in der zuständigen Gastzelle erlauben, wobei das rekombinierende Gen der Kontrolle eines äußeren Promoters untersteht, der sich vom vorherigen unterscheidet und ebenfalls von den Polymerasen der zuständigen Gastzelle erkannt werden kann, und dieses unter Bedingungen, die die Transcription der Zusammenstellung der oben beschriebenen Elemente in eine entgegengesetzte Richtung (Gegenrichtung) zu der von dem inneren Promoter vorgegebenen Richtung erlauben.

10. Verfahren nach Anspruch 9, **dadurch gekennzeichnet**, daß die Sequenz, die die erste oben genannte Transription blockieren kann, aus einer Stelle von Polyadenylation besteht.

11. Verfahren zur Herstellung der ADN eines defektiven rekombinierenden Retrovirus, abgeleitet aus einem wilden oder natürlichen Retrovirus, der fähig ist, eine zuständige eukariotische Gastzelle, insbesondere eine Mammifere, zu infizieren, wobei diese ADN diejenigen Abschnitte enthält, die denen entsprechen, die in diesem Retrovirus als "cis" des wilden Virus tätig sind und normalerweise die Umhüllung des Retrovirus, seine reverse Transcriptase in ein ADN-Molekül und seine Einbindung in das Genom der Gestzelle gewährleisten, ebenso wie die Stelle der Polyadenylation dieses ADN, **dadurch gekennzeichnet**, daß man ein Gebiet entfernt, das wenigstens einem Teil, wenn nicht der Gesamtheit der Kodiersequenzen für die Virusproteine des wilden oder natürlichen Virus entspricht, wobei dieser entfernte Teil wichtig genug ist, um die Replikation des defektiven Retrovirus zu verhindern, und geeignet ist hergestellt zu werden in einer entsprechenden zuständigen Gastzelle, und weiter dadurch gekennzeichnet ist, daß dieses Gebiet ersetzt wird durch das rekombinierte Gen, hergestellt nach Anspruch 9, unter der Kontrolle eines äußeren bestimmten Promoters, der in der ADN des rekombiierenden Retorvirus eingeschlossen ist, wobei die gegenseitige Anordnung dieses äußeren bestimmten Promoters und des rekombinierenden Gens sowie dessen Orientierung so gewählt sind, daß die Transcription dieses rekombinierenden Gens unter der Kontrolle dieses äußeren Promoters in der erwähnten umgekehrten Richtung durchgeführt werden kann in den Gastzellen, die von dem so zusammengesetzten defektiven rekombinierenden Retrovirus infiziert werden können.

12. Verfahren nach Anspruch 11, **dadurch gekennzeichnet**, daß der bestimmte äußere Promoter der Promoter aus dem Gebiet LTR5 des Retrovirus ist.

13. Verfahren zur Herstellung eines rekombinierenden retroviralen defektives ADN, dessen Genom nacheinander umfaßt:
- das Gebiet LTR5 des jeweiligen wilden Retrovirus, gefolgt von dem Gebiet der Umhüllung
- die Anfangsstelle des ARN, das den Virusproteinen zugeordnet ist, die normalerweise durch die Gene qaq, pol und env verschlüsselt sind
- das rekombinierende Gen hergestellt nach dem Verfahren der Ansprüche 9 oder 10 in der oben beschriebenen Ausrichtung, an Ort und Steile des vollständigen oder unvollständigen Gebiets der Retrovirus, das die Gene qaq, pol und env umfaßt, wobei dieses rekombinierende Gen der Kontrolle eines geeigneten äußeren Promoters untersteht, der aus dem Gebiet LTR5 selbst hervorgeht, und
- das Gebiet LTR3, das zusammenfällt mit der Stelle der Polyadenylatopn des ADN des zugehörigen wilden Retrovirus**, dadurch gekennzeichnet**, daß man mit einem Vektor, der das retrovirale ADN enthält, das Gebiet, das die Gene qaq, pol und env enthält, ganz oder teilweise entfernt und danach das rekombinierende Gen in dieses Gebiet einsetzt wobei diese Substitution aufeine Weise durchgeführt wird, die das rekombinierende Gen der Kontrolle eines geeigneten äußeren Promoters unterstellt.

14. Verfahren zur Einlagerung einer ein bestimmtes Polypetid verschlüsselnden Nukleotidsequenz in das Genom von Zellen einer Zellkultur, die durch den natürlichen Retrovirus, der dem rekombinierenden defektiven Retrovirus nach einem der Ansprüche 6 - 8 entspricht, infizierbar ist,, **dadurch gekennzeichnet**, daß der Kontakt dieser Zellkultur mit dem defektiven rekombinerenden Retrovirus, in dem obige Kodiersequenz obiger Nukleotidsequenz entspricht, die das bestimmte Polypeptid verschlüsselt, unter Bedingungen erfolgt, die die Infektion dieser Zellen durch den defektiven rekombinierenden Retrovirus ermöglichen, wobei die Zellen isoliert werden, in denen die Kodiersequenz ausgebildet ist.

15. Verfahren zur Herstellung eines defektiven rekombinierenden Retrovirus nach einem der Ansprüche 6 - 8, das umfaßt:
- die Transfektion eines Hilfsstamms von Zellen mit einem Vektor, der die ADN des defektiven rekombinierenden Retrovirus umfaßt, der nach einem der Ansprüche 11 - 13 hergestellt ist, wobei dieser Hilfsstamm von Zellen eine Nukleotidsequenz, eingelagert in sein eigenes ADN im Genom (im folgenden als "Vervollständigungssequenz" bezeichnet) enthält, die fähig bei Abwesenheit im defektiven rekombinierenden Vektor Kodiersequenzen bereitzustellen, die Virusproteine verschlüsseln, die die Verbreitung des Retrovirus in den Zellen ermöglichen, die er infizieren kann,
- die Kultur des Hilfstamms von Zellen, die so transfiziert ist in Bedingungen, die die Replikation des rekombinierenden Retrovirus in situ ermöglichen, und zwar wegen der Vervollständigung während der Transcription des Virusproteine, die durch obige Vervollständigungssequenz verschlüsselt werden,
- die Wiederverwertung des defektiven rekombinierenden Virus, der im Überstand der Kultur des angesprochenen Hilfsstamms von Zellen erzeugt wird.

16. Verfahren nach Anspruch 15, **dadurch gekennzeichnet**, daß die Vervollständigungssequenz insbesondere den Teil der im Genom des zugehörigen wilden Retrovirus vorhandenen ADN enthält, der besonders die Virusproteine verschlüsselt besonders die Gene qaq, pol und env des wilden zugehörigen Retrovirus, die aus dem defektiven rekombinierenden Retrovirus entfernt sind.
